# EUROPEAN PATENT APPLICATION

(11) **EP 3 854 359 A1**
(43) Date of publication of application: **28.07.2021**
(21) Application number: 21153597.6
(22) Date of filing: 26.01.2021
(51) Int. Cl.: A61F 7/00

(54) **TISSUE ERADICATION METHODS AND SYSTEMS**

(30) Priority: 27.01.2020 US 202062966110 P
(71) Applicant: Wall, Jr., Simeon, Shreveport, LA 71105 (US)
(72) Inventor: Wall, Jr., Simeon, Shreveport, LA 71105 (US)
(74) Representative: Appleyard Lees IP LLP

(57) **Abstract**

Methods for at least partially eradicating a target tissue by apoptosis may include reducing the temperature of a device shaft on a tissue eradication device to a target temperature, vibrating the device shaft and inducing apoptosis in the target tissue by contacting the target tissue with the device shaft or injecting a cold fluid from the cannulated device shaft of the tissue eradication device into the target tissue. Tissue eradication systems are also disclosed.

## Description

### FIELD

Illustrative embodiments of the disclosure generally relate to body tissue shaping systems and methods, such as non-therapeutic (such as cosmetic) methods. More particularly, illustrative embodiments of the disclosure relate to tissue apoptosis methods and systems which facilitate at least partial eradication of adipose or other target tissue by apoptosis of the tissue in body shaping or other applications.

### SUMMARY

Illustrative embodiments of the disclosure are generally directed to methods for at least partially eradicating a target tissue by apoptosis. An illustrative embodiment of the tissue eradication methods may include reducing the temperature of a device shaft on a tissue eradication device to a target temperature and inducing apoptosis in the target tissue by contacting the target tissue with the device shaft or injecting a cold fluid from a cannulated device shaft of the tissue eradication device into the target tissue. In some embodiments, the device shaft may be vibrated as the device shaft is placed into contact with the target tissue or as the cold fluid is injected from the cannulated device shaft into the target tissue.

Illustrative embodiments of the disclosure are further generally directed to tissue eradication systems suitable for at least partially eradicating a target tissue by apoptosis. An illustrative embodiment of the tissue eradication systems may include a tissue eradication device. The tissue eradication device may include a gripping portion. A cannulated device shaft may extend from the gripping portion. A cold temperature source may include a pump disposed in fluid communication with the cannulated device shaft. A cold fluid container may be disposed in fluid communication with the pump. The cold fluid container may be configured to contain a supply of cold fluid. A fluid temperature control may be disposed in thermal communication with the cold fluid container.

In some embodiments, the tissue eradication systems may include a tissue eradication device having a gripping portion and a device shaft extending from the gripping portion. A cold temperature source may be disposed in thermal contact with the device shaft. A vibratory motor may be disposed in contact with at least one of the gripping portion and the device shaft for vibration of the device shaft.

The present invention provides a tissue eradication system for at least partially eradicating a target tissue, comprising:
a tissue eradication device including:
a gripping portion;
a cannulated device shaft extending from the gripping portion; and
a cold temperature source including:
   a pump disposed in fluid communication with the cannulated device shaft;
   a cold fluid container disposed in fluid communication with the pump, the cold fluid container configured to contain a supply of cold fluid; and
   a fluid temperature control disposed in thermal communication with the cold fluid container.

The tissue eradication system may further comprise a vibratory motor disposed in contact with at least one of the gripping portion and the cannulated device shaft for vibration of the cannulated device shaft.

The cannulated device shaft may comprise an elongated cannula wall, a cannula lumen formed by the cannula wall, a distal shaft end having a shaft tip, a plurality of tissue separating members extending outwardly from and in spanning relationship between the cannula wall and the distal shaft end and a plurality of fluid flow spaces formed by and between the plurality of tissue separating members and communicating with the cannula lumen.

The present invention provides a tissue eradication system for at least partially eradicating a target tissue, comprising:
a tissue eradication device including:
a gripping portion;
a device shaft extending from the gripping portion;
a cold temperature source disposed in thermal contact with the device shaft, the cold temperature source configured to reduce the temperature of the device shaft to a selected target temperature or target temperature range; and
a vibratory motor disposed in contact with at least one of the gripping portion and the device shaft for vibration of the device shaft.

The cold temperature source may comprise a cold fluid container configured to contain a supply of cold fluid.

The present invention provides a tissue eradication method for at least partially eradicating a target tissue, comprising:
reducing the temperature of a cold fluid to a target temperature or target temperature range;
inserting a cannulated device shaft of a tissue eradication device into the target tissue; and
inducing apoptosis in the target tissue by injecting the cold fluid from the cannulated device shaft of the tissue eradication device into the target tissue.

The tissue eradication may further comprise vibrating the cannulated device shaft of the tissue eradication device simultaneous with injecting the cold fluid from the cannulated device shaft of the tissue eradication device into the target tissue.

Vibrating the cannulated device shaft of the tissue eradication device may comprise vibrating the cannulated device shaft at a vibration or reciprocation frequency within a range of about 2,000-10,000 cycles/min.

Vibrating the cannulated device shaft at the vibration or reciprocation frequency within the range of about 2,000-10,000 cycles/min may comprise vibrating the cannulated device shaft at the vibration or reciprocation frequency of about 7,000 cycles/min.

Reducing the temperature of the cold fluid to the target temperature or target temperature range may comprise reducing the temperature of the cold fluid to the target temperature or target temperature range of from about -21.12° C to about 0°C.

Injecting the cold fluid from the cannulated device shaft of the tissue eradication device into the target tissue may comprise injecting tumescent fluid, hypertonic saline solution, epinephrine solution, liposuction infiltration fluid or lactated ringer's solution from the cannulated device shaft into the target tissue.

Inserting the cannulated device shaft of the tissue eradication device into the target tissue may comprise inserting the cannula device shaft having an expanded basket cannula with a plurality of outwardly-extending tissue separation members into the target tissue.

The tissue eradication method may further comprise providing a pump in fluid communication with the cannulated device shaft of the tissue eradication device, providing a cold fluid container in fluid communication with the pump and providing a supply of the cold fluid in the cold fluid container, and wherein injecting the cold fluid from the cannulated device shaft of the tissue eradication device into the target tissue comprises pumping the cold fluid from the cold fluid container through the cannulated device shaft into the target tissue.

The present invention provides a tissue eradication method for at least partially eradicating a target tissue, comprising:
reducing the temperature of a device shaft on a tissue eradication device to a target temperature or target temperature range;
vibrating the device shaft; and
inducing apoptosis in the target tissue by contacting the target tissue with the device shaft.

Vibrating the device shaft may comprise vibrating the device shaft at a vibration or reciprocation frequency within a range of about 2,000-10,000 cycles/min.

Vibrating the device shaft at the vibration or reciprocation frequency within the range of about 2,000-10,000 cycles/min may comprise vibrating the device shaft at the vibration or reciprocation frequency of about 7,000 cycles/min.

Reducing the temperature of the device shaft on the tissue eradication device to the target temperature or target temperature range may comprise reducing the temperature of the device shaft on the tissue eradication device to the target temperature range of from about -21.12° C to about 0°C.

Reducing the temperature of the device shaft on the tissue eradication device to the target temperature or target temperature range may comprise placing at least one cold temperature source in thermal communication with the device shaft.

Placing at least one cold temperature source in thermal communication with the device shaft may comprise placing a cold fluid in thermal communication with the device shaft.

Reducing the temperature of the device shaft on the tissue eradication device to the target temperature or target temperature range may comprise reducing the temperature of the device shaft having an expanded basket cannula with a plurality of outwardly-extending tissue separation members to the target temperature or target temperature range.

The system and methods of the present invention provide non-therapeutic (for example cosmetic) effects to the patient. In other words, the method of the present invention is a non-therapeutic, i.e. a cosmetic, method. The system and method act to improve the bodily appearance of a patient. Thus, references herein to shaping the body surface of a patient are to shaping so as to improve the bodily appearance of the patient.

The present invention also provides the use of a tissue eradication system according to the present invention to at least partially eradicate a target tissue.

The present invention also provides the use of a tissue eradication system according to the present invention to at least partially eradicate a target tissue by inducing apoptosis in the target tissue by injecting cold fluid into the target tissue, for example by injecting cold fluid from the cannulated device shaft of the tissue eradication device.

The present invention also provides the use of a system according to the present invention to volumetrically reduce and smoothen the contour of the skin of a patient, for example as a cosmetic procedure.

The present invention also provides the use of a system according to the present invention to eliminate or reduce the quantity or volume of adipose cells in an adipose tissue layer at a treatment site in the skin of a patient, for example as a cosmetic procedure.

The present invention also provides the use of a system according to the present invention to rectify contour irregularities in the buttocks, hip and/or lower back of a patient, for example as a cosmetic procedure.

### BRIEF DESCRIPTION OF THE DRAWINGS

Illustrative embodiments of the disclosure will now be described, by way of example, with reference to the accompanying drawings, in which:
FIG. 1 is a block diagram of an illustrative embodiment of the tissue eradication systems which is suitable for implementation of the tissue eradication methods of the disclosure;
FIG. 2 is a longitudinal sectional view of a segment of a typical cannulated device shaft on a tissue eradication device of the tissue eradication system illustrated in FIG. 1, more particularly illustrating a typical discharge path of cold fluid through and from the cannulated device shaft in exemplary application of the tissue eradication methods;
FIG. 3 is a cross-sectional view of the cannulated device shaft and tissue separating members on the device shaft of the tissue eradication device, taken along section lines 3-3 in FIG. 2, with the typical discharge path of the cold fluid;
FIG. 4 is a cross-sectional view of a section of a patient's skin with an incision extending through the epidermis and the underlying dermis and into a target adipose tissue layer preparatory to inserting the cannulated device shaft through the incision into the adipose tissue layer in typical implementation of the tissue eradication methods;
FIG. 5 is a cross-sectional view of the section of the patient's skin with the cannulated device shaft inserted in place in the incision and the adipose tissue layer and more particularly illustrating typical discharge of the cold fluid through the cannulated device shaft into the target adipose tissue;
FIG. 6 is a cross-sectional view of the section of the patient's skin after continued injection of the cold fluid through the cannulated device shaft into the adipose tissue;
FIG. 7 is a cross-sectional view of the treated section of the patient's skin upon conclusion of the tissue apoptosis method;
FIG. 8 is a flow diagram of an illustrative embodiment of the tissue eradication methods carried out using the tissue eradication system illustrated in FIG. 1;
FIG. 9 is a block diagram of an alternative illustrative embodiment of the tissue eradication systems which is suitable for implementation of the tissue eradication methods of the disclosure;
FIG. 10 is a longitudinal sectional view of a segment of a typical device shaft on a tissue eradication device of the tissue eradication system illustrated in FIG. 9;
FIG. 11 is a cross-sectional view of the device shaft and tissue separating members on the device shaft of the tissue eradication device illustrated in FIG. 10;
FIG. 12 is a cross-sectional view of a section of a patient's skin with an incision extending through the epidermis and the underlying dermis and into the target adipose tissue layer preparatory to inserting the device shaft through the incision into the adipose tissue layer in typical implementation of the tissue eradication methods;
FIG. 13 is a cross-sectional view of the section of the patient's skin with the device shaft inserted in place in the incision and into the target adipose tissue;
FIG. 14 is a cross-sectional view of the section of the patient's skin after continued contact of the device shaft with the adipose tissue;
FIG. 15 is a cross-sectional view of the treated section of the patient's skin upon conclusion of the tissue eradication method; and
FIG. 16 is a flow diagram of an alternative illustrative embodiment of the tissue eradication methods using the tissue eradication system illustrated in FIG. 9.

### DETAILED DESCRIPTION

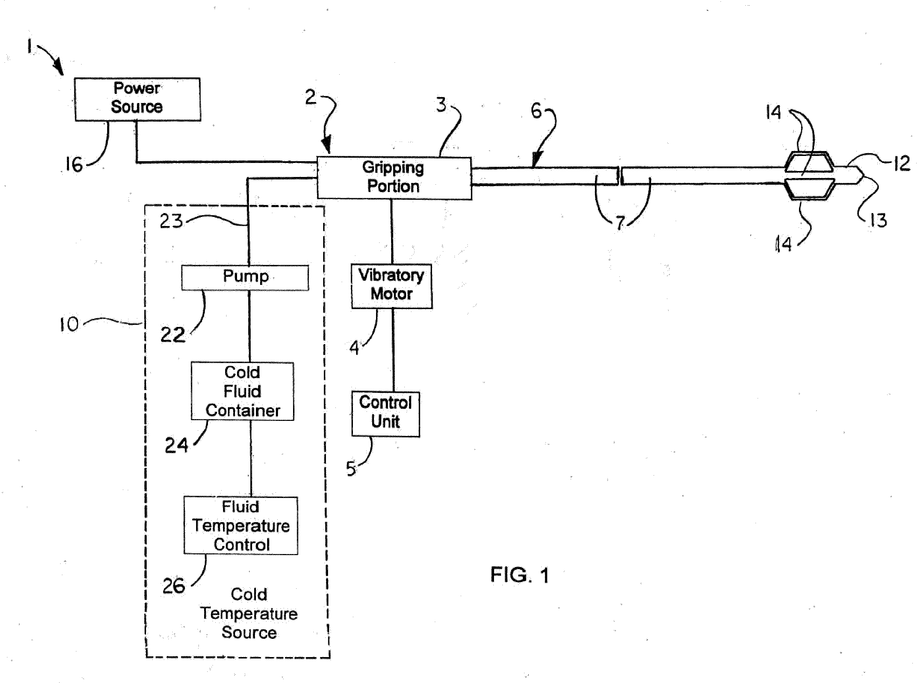

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. For purposes of description herein, the terms "upper", "lower", "left", "rear", "right", "front", "vertical", "horizontal", and derivatives thereof shall relate to the invention as oriented in FIG. 1. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

Referring initially to FIG. 1 of the drawings, an illustrative embodiment of the tissue eradication systems, hereinafter system, of the disclosure which is suitable for implementation of the tissue eradication methods, hereinafter method, of the disclosure is generally indicated by reference numeral 1. The system 1 may include a tissue eradication device 2. For example and without limitation, in some applications, the tissue eradication device 2 may have a design which is the same as or similar to that of the tissue injection device which is disclosed in U.S Pub. No. 2013/0310749, which is hereby incorporated by reference herein in its entirety. The tissue eradication device 2 may include a gripping portion 3. In some embodiments, at least one vibratory motor 4 may engage the gripping portion 3 for vibration. The vibratory motor 4 may be electric, hydraulic and/or pneumatic. For example and without limitation, in some applications, the vibratory motor 4 may have a design which is the same as or similar to those described with respect to the electric vibrational apparatuses or the pneumatic vibrational apparatuses which are disclosed in U.S. Pat. No. 10,173,000, which patent is hereby incorporated by reference herein in its entirety.

A control unit 5 may operationally interface with the vibratory motor 4. The control unit 5 may include but is not limited to buttons, switches, slides, triggers, touch screens and/or other input control devices which are operable to control various operational parameters such as the vibration frequency and/or the vibration amplitude, for example and without limitation, of the vibratory motor 4 according to the knowledge of those skilled in the art. The control unit 5 may additionally include a display which indicates such data as status information and/or vibrational intensity (rate and/or amplitude) of the vibratory motor 4, for example and without limitation.

At least one power source 16 may operably interface with the gripping portion 3. The power source 16 may include at least one battery and/or at least one electrical wall outlet, for example and without limitation.

An elongated, cannulated device shaft 6 may extend from the gripping portion 3. The cannulated device shaft 6 may be coupled to the gripping portion 3 using a locked thread connector such as a Luer lock connector, for example and without limitation. In some embodiments of the tissue eradication device 2, the vibratory motor 4 may engage the cannulated device shaft 6 for oscillation or reciprocation directly or indirectly through the gripping portion 3.

The cannulated device shaft 6 may include a trocar or any other type of needle or cannula which is typically used or suitable for fat or tissue injection procedures or the like and may have a distal shaft end 12 with a sharpened, pointed or tapered shaft tip 13. In some applications, the cannulated device shaft 6 may include an exploded-tip or expanded basket cannula such as those which are described in U.S. Pat. No. 10,188,280, which patent is hereby incorporated by reference herein in its entirety. A non-limiting example of a cannulated device shaft 6 which is suitable for implementation of the methods is a 4-5 mm exploded tip cannula available from Surgistem Technologies of Boston, MA. In some applications, the cannulated device shaft 6 may be angled to facilitate further "reach" of the cannulated device shaft 6 during the apoptosis procedure, resulting in more extensive and thorough eradication of the target tissue.

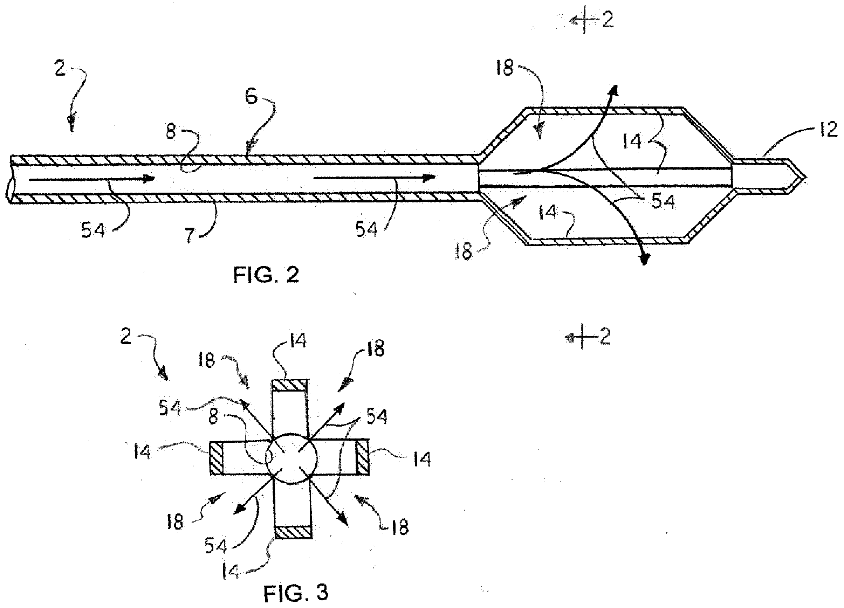

As illustrated in FIGS. 2 and 3, in some embodiments, the exploded tip cannulated device shaft 6 may have an elongated cannula wall 7 which surrounds a cannula lumen 8. A plurality of tissue separating members 14 may extend outwardly from and in spanning relationship between the cannula wall 7 and the distal shaft end 12. Fluid flow spaces 18 which communicate with the cannula lumen 8 may be formed by and between the tissue separating members 14.

As further illustrated in FIG. 1, a cold temperature source 10 may be disposed in thermal contact with the cannulated device shaft 6 of the tissue eradication device 2. In some embodiments, the cold temperature source 10 may include a pump 22 which may be disposed in fluid communication with the gripping portion 3 such as through a suitable pump tubing 23. At least one cold fluid canister or container 24 may be disposed in fluid communication with the pump 22. The cold fluid container 24 may be sized and configured to contain a supply of cold fluid 54 (FIG. 2). The cold fluid 54 may include any type of liquid or other fluid the temperature of which can be reduced to a selected target temperature or target temperature range for contact with and apoptosis of adipose or other target tissue, as will be hereinafter described. Non-limiting examples of fluids which are suitable for the cold fluid 54 include but are not limited to tumescent fluid, hypertonic saline solution, epinephrine solution, liposuction infiltration fluid and lactated ringer's solution. In some applications, the ringer's solution may include about 1-1,000,000 epinephrine solution by volume.

The pump 22 of the cold temperature source 10 may include a peristaltic pump or any type of positive displacement pump or other automated and/or manual mechanical delivery system which is known by those skilled in the art and is operable to pump the cold fluid 54 from the cold fluid container 24 through the gripping portion 3 and the cannula lumen 8 of the cannulated device shaft 6, respectively. For example and without limitation, in some applications, the pump 22 may include a syringe such as a Toomey syringe known by those skilled in the art. In some embodiments, the pump 22 and the cold fluid container 24 may be combined in the same device or apparatus.

A fluid temperature control 26 may be disposed in thermal communication with the cold fluid container 24. The fluid temperature control 26 may include a refrigeration system and/or other type of system which is known by those skilled in the art and configured to reduce the temperature of the cold fluid container 24 or the cold fluid 54 in the cold fluid container 24 to a selected target temperature or target temperature range which is sufficient to induce apoptosis in the target tissue. The fluid temperature control 26 may include any source of cold temperature which is known by those skilled in the art and suitable for the purpose. For example and without limitation, in some embodiments, the cold temperature source may utilize a source of refrigerant (not illustrated) to achieve and maintain the cold fluid container 24 or the cold fluid 54 therein at the target temperature or within the target temperature range via compression and evaporation of the refrigerant according to conventional methods known by those skilled in the art. In some non-limiting applications, the fluid temperature control 26 may be suitably configured to reduce the temperature of the cold fluid 54 to a target temperature or target temperature range of from about -21.12° C to about 0°C.

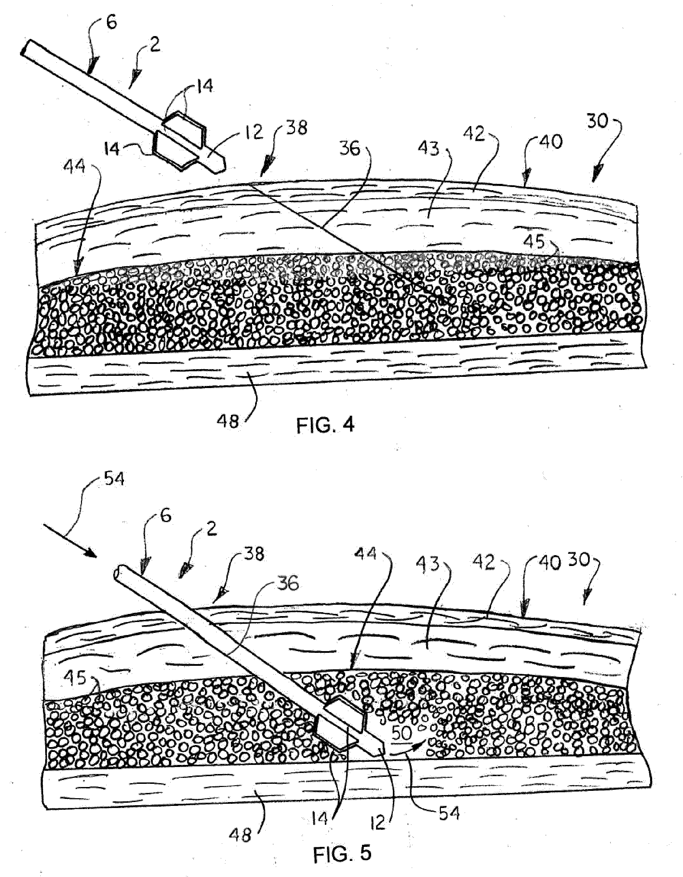

Referring next to FIGS. 4-7 of the drawings, in typical implementation of the tissue eradication methods, the system 1 (FIG. 1) may be used to volumetrically reduce and smoothen the contour of the skin 40 of a patient 30 as part of a cosmetic procedure. For example and without limitation, in some applications, the method may be implemented to rectify contour irregularities in the buttocks, hip and/or lower back (not illustrated) of the patient 30. As illustrated in FIG. 4, the skin 40 of the patient 30 has an epidermis 42 and an underlying dermis 43. An adipose tissue layer 44 having adipose cells 45 underlies the subdermal space beneath the dermis 43. A muscle layer 48 underlies the adipose tissue layer 44.

In the non-limiting example illustrated in FIGS. 4-7, the method may be implemented in a cosmetic procedure to eliminate or reduce the quantity or volume of adipose cells 45 in an adipose tissue layer 44 at a treatment site 38 in the skin 40 of the patient 30. Accordingly, a supply of the cold fluid 54 may be placed in the cold fluid container 24 (FIG. 1) of the system 1. The fluid temperature control 26 of the cold temperature source 10 may be operated to reduce the temperature of the cold fluid 54 to the selected target temperature or target temperature range. For example and without limitation, in some applications, the temperature of the cold fluid 54 may be reduced to a target temperature or target temperature range of from about -21.12° C to about 0°C, for example and without limitation.

As illustrated in FIG. 4, as the patient 30 typically lies in a supine or lateral position, an incision 36 may be formed in the skin 40 at or adjacent to the treatment site 38. The incision 36 may extend through the epidermis 42 and dermis 43 and into the underlying adipose tissue layer 44 of the skin 40.

As illustrated in FIG. 5, the cannulated device shaft 6 of the tissue eradication device 2 may next be inserted through the incision 36 until the distal shaft end 12 and tissue separating members 14 of the cannulated device shaft 6 are disposed within the adipose tissue layer 44. In some applications, the tissue separating members 14 on the cannulated device shaft 6 may separate adipose cells 45 in the adipose tissue layer 44.

Responsive to subsequent operation of the pump 22 (FIG. 1) of the system 1 in the suction phase, the cold fluid 54 may be drawn from the cold fluid container 24 through the pump tubing 23 and the gripping portion 3 and cannulated device shaft 6, respectively, of the tissue eradication device 2. As further illustrated in FIG. 5, the cold fluid 54 may be discharged from the cannulated device shaft 6 into the adipose tissue layer 44. The supercooled cold fluid 54 contacts and induces apoptosis in the adipose cells 45, forming a tissue eradication space 50 in the adipose tissue layer 44.

In some applications, the vibratory motor 4 (FIG. 1) of the tissue eradication device 2 may simultaneously be operated to vibrate or reciprocate the cannulated device shaft 6 and achieve enhanced dispersal and homogenous distribution of the cold fluid 54 within the adipose tissue layer 44 and corresponding enlargement of the tissue eradication space 50. The various operational parameters of the vibratory motor 4, such as the vibration frequency and/or the vibration amplitude, for example and without limitation, may be selected by input from the control unit 5. A typical vibration or reciprocation frequency for the cannulated device shaft 6 may fall within the range of about 2,000-10,000 cycles/min, with a typical frequency of about 7,000 cycles/min.

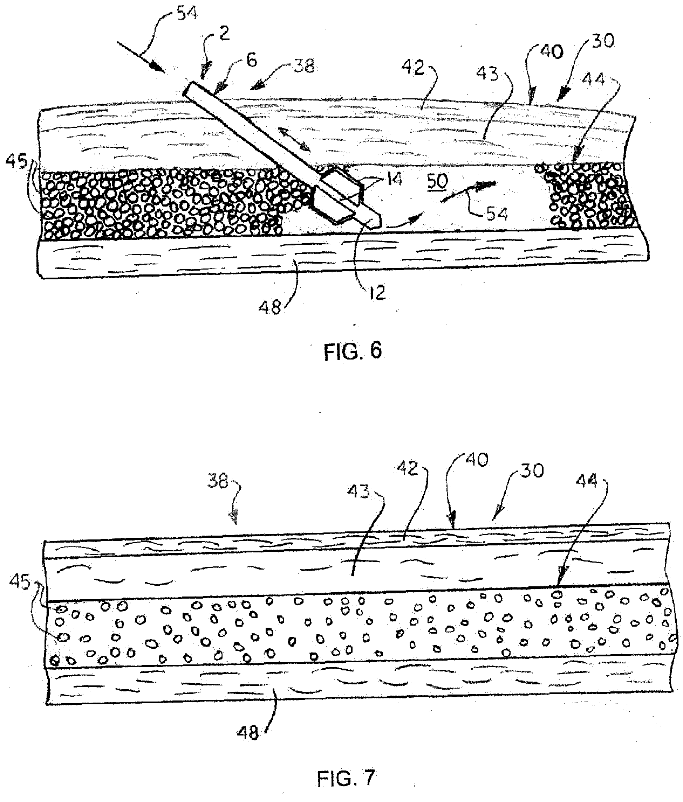

As illustrated in FIG. 6, continued operation of the pump 22 may facilitate further discharge of the cold fluid 54 from the cannulated device shaft 6 into the adipose tissue layer 44, inducing apoptosis in an additional number, quantity or volume of adipose cells 45 which is proportional to the quantity or volume of cold fluid 54 and correspondingly enlarging the tissue eradication space 50. The quantity or volume of cold fluid 54 which is discharged from the cannulated device shaft 6 into the adipose tissue layer 44 may be selected depending on the quantity or volume of adipose or tissue which is to be at least partially eradicated from the adipose tissue layer 44 in order to achieve a desired reduction of adipose tissue and cosmetic effect in the skin 40 of the patient 30.

At the conclusion of the tissue eradication procedure, operation of the pump 22 may be terminated. The cannulated device shaft 6 of the tissue eradication device 2 may be removed from the incision 36 and the incision 36 closed. As illustrated in FIG. 7, the adipose cells 45 which remain in the adipose tissue layer 44 may migrate and even out through the adipose tissue layer 44, resulting in a volumetric reduction in the thickness of the skin 40 at and adjacent to the treatment site 38.

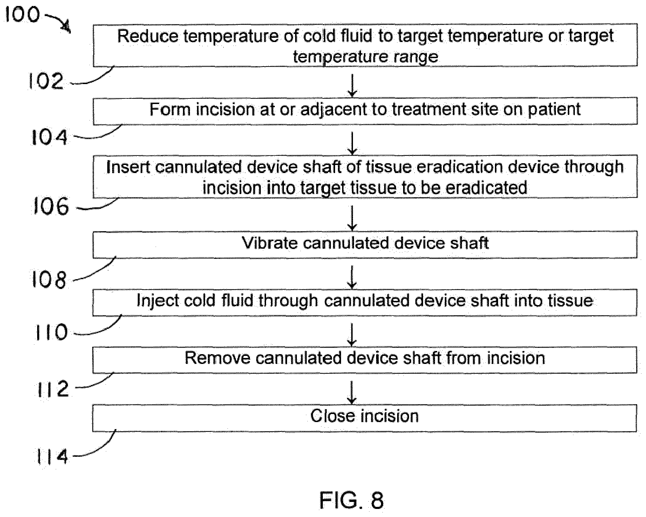

Referring next to FIG. 8 of the drawings, a flow diagram of an illustrative embodiment of the tissue eradication methods is generally indicated by reference numeral 100. At Step 102, the temperature of a cold fluid may be reduced to a selected target temperature or target temperature range which is sufficient to induce apoptosis in adipose tissue or other target tissue. In some applications, the target temperature may fall within a target temperature range of from about - 21.12° C to about 0°C.

At Step 104, an incision may be formed in the skin of a patient at or adjacent to a treatment site on the skin to access a target tissue. The incision may extend through the epidermis and dermis and into the underlying adipose tissue layer or other target tissue.

At Step 106, the cannulated device shaft of a tissue eradication device may be inserted through the incision and into the target tissue to be at least partially eradicated. In some applications, the target tissue may include adipose tissue. In other applications, the target tissue may include any other target tissue which is to be volumetrically reduced.

At Step 108, the cannulated device shaft may be vibrated. A typical, non-limiting vibration or reciprocation frequency for the cannulated device shaft may fall within the range of about 2,000-10,000 cycles/min, with a typical frequency of about 7,000 cycles/min.

At Step 110, the cold fluid may be injected through the cannulated device shaft of the tissue eradication device into the target tissue. The quantity or volume of cold fluid which is injected into the target tissue may be selected depending on the quantity or volume of the target tissue which is to be at least partially eradicated from the target tissue layer such as, for example and without limitation, to achieve a desired reduction of adipose tissue and cosmetic effect in the skin of the patient in some applications.

At Step 112, the cannulated device shaft may be removed from the incision.

At Step 114, the incision may be closed.

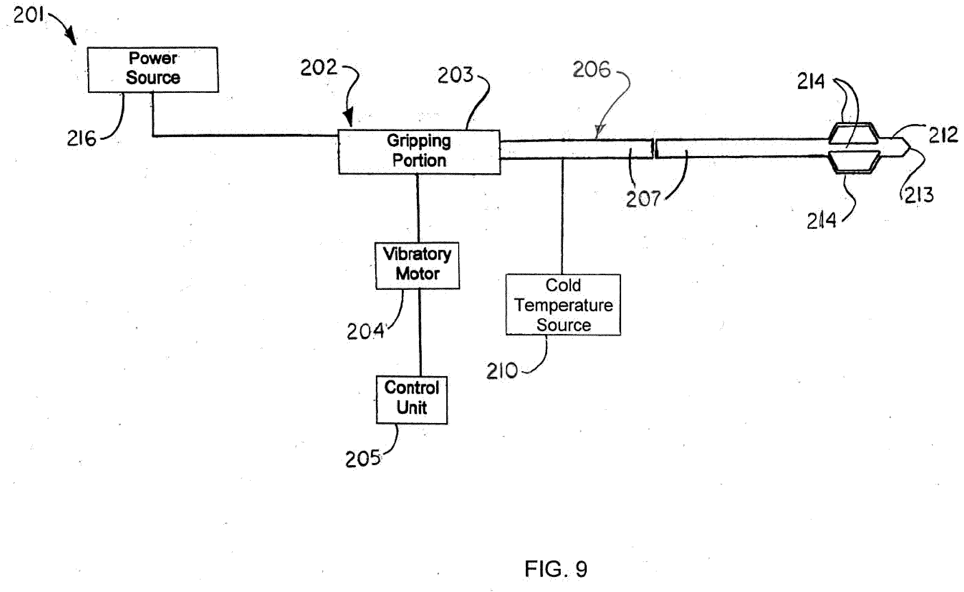

Referring next to FIG. 9 of the drawings, an alternative illustrative embodiment of the tissue eradication systems, hereinafter system, of the disclosure which is suitable for implementation of the tissue eradication methods, hereinafter method, of the disclosure is generally indicated by reference numeral 201. In the system 201, elements which are analogous to the respective elements of the system 1 that was heretofore described with respect to FIG. 1 are designated by the same respective numerals in the 201-299 series in FIG. 9. The tissue eradication device 202 of the system 201 may include a gripping portion 203. In some embodiments, at least one vibratory motor 204 may engage the gripping portion 203 for vibration. The vibratory motor 204 may be electric, hydraulic and/or pneumatic. For example and without limitation, in some applications, the vibratory motor 204 may have a design which is the same as or similar to those described with respect to the electric vibrational apparatuses or the pneumatic vibrational apparatuses which are disclosed in U.S. Pat. No. 10,173,000, which patent is hereby incorporated by reference herein in its entirety.

A control unit 205 may operationally interface with the vibratory motor 204. The control unit 205 may include but is not limited to buttons, switches, slides, triggers, touch screens and/or other input control devices which are operable to control various operational parameters such as the vibration frequency and/or the vibration amplitude, for example and without limitation, of the vibratory motor 204 according to the knowledge of those skilled in the art. The control unit 205 may additionally include a display which indicates such data as status information and/or vibrational intensity (rate and/or amplitude) of the vibratory motor 204, for example and without limitation.

At least one power source 216 may operably interface with the gripping portion 203. The power source 216 may include at least one battery and/or at least one electrical wall outlet, for example and without limitation.

An elongated device shaft 206 may extend from the gripping portion 203. The device shaft 206 may be fabricated of steel and/or other thermally-conductive metals or materials. The device shaft 206 may have a main shaft segment 207 which may be coupled to the gripping portion 203 using a locked thread connector such as a Luer lock connector, for example and without limitation. In some embodiments of the tissue eradication device 202, the vibratory motor 204 may engage the device shaft 206 for oscillation or reciprocation directly or indirectly through the gripping portion 203.

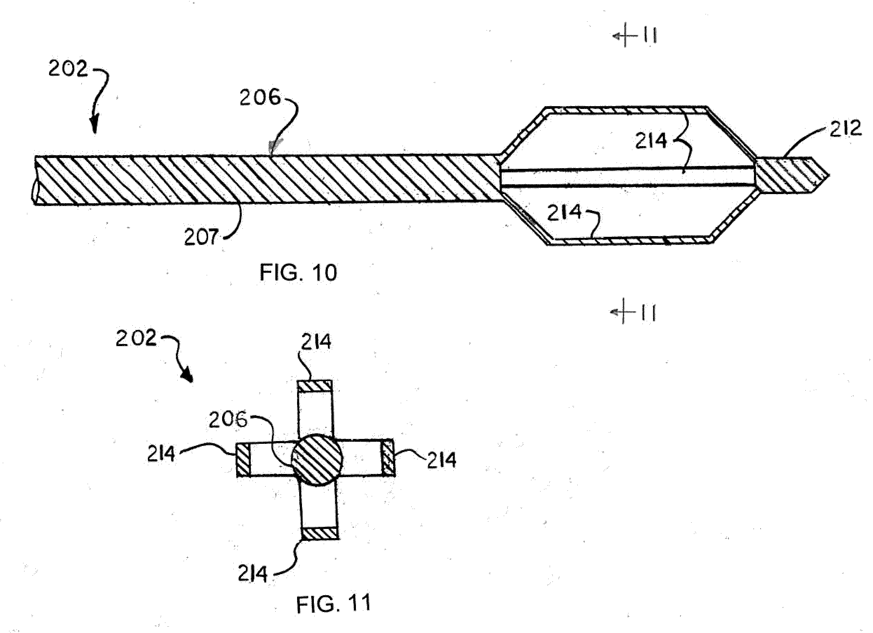

The device shaft 206 may have a distal shaft end 212 with a sharpened, pointed or tapered shaft tip 213. In some applications, the device shaft 206 may include an exploded-tip or expanded basket cannula such as those which are described in U.S. Pat. No. 10,188,280, which patent is hereby incorporated by reference herein in its entirety. In some applications, the device shaft 206 may be angled to facilitate further "reach" of the device shaft 206 during the apoptosis procedure, resulting in more extensive and thorough eradication of the target tissue. As illustrated in FIGS. 10 and 11, in some embodiments, a plurality of tissue separating members 214 may extend outwardly from and in spanning relationship between the main shaft segment 207 of the device shaft 206 and the distal shaft end 212.

As further illustrated in FIG. 9, at least one cold temperature source 210 may be disposed in thermal communication with the device shaft 206. In some embodiments, the cold temperature source 210 may include a cold fluid (not illustrated). The cold fluid may include any type of liquid or other fluid the temperature of which can be reduced to a selected target temperature or target temperature range for contact with the device shaft 206 of the tissue eradication device 202 and reducing the temperature of the device shaft 206 typically by conduction. Non-limiting examples of cold fluids which are suitable for the purpose include but are not limited to tumescent fluid, hypertonic saline solution, epinephrine solution, liposuction infiltration fluid and lactated ringer's solution. In some embodiments, the cold temperature source 210 may include cold fluid container (not illustrated) which contains the cold fluid as well as the necessary conduits and/or pumps which facilitate thermal application of the cold fluid to the device shaft 206 to reduce the temperature of the device shaft 206 to the selected target temperature or target temperature range. The cold temperature source 210 may include a fluid or other temperature control (not illustrated) to facilitate selective control of the temperature of the cold temperature source 210 according to the knowledge of those skilled in the art.

The cold temperature source 210 may include a refrigeration system and/or other type of system which is known by those skilled in the art and configured to reduce the temperature of the device shaft 206 to the selected target temperature or target temperature range which is sufficient to induce apoptosis in the target tissue. The cold temperature source 210 may include any source of cold temperature which is known by those skilled in the art and suitable for the purpose. For example and without limitation, in some embodiments, the cold temperature source 210 may utilize a source of refrigerant (not illustrated) to achieve and maintain the cold fluid at the target temperature or within the target temperature range via compression and evaporation of the refrigerant according to conventional methods known by those skilled in the art. In some non-limiting applications, the cold temperature source 210 may be suitably configured to reduce the temperature of the cold fluid to a target temperature or target temperature range of from about - 21.12° C to about 0°C.

As illustrated in FIGS. 10 and 11, in some embodiments, the device shaft 206 of the tissue eradication device 202 may be substantially solid or semisolid in construction. Accordingly, the cold temperature source 210 (FIG. 9) may include a cold fluid which contacts and cools the device shaft 206 by conduction. In other embodiments, at least one fluid flow channel (not illustrated) may be provided in the device shaft 206. The cold fluid may flow through the fluid flow channel in a closed loop and cool the device shaft 206 typically by conduction. In still other embodiments, the cold temperature source 210 may reduce and maintain the temperature of the device shaft 206 at or within the target temperature range using any of a variety of other techniques or methods known by those skilled in the art.

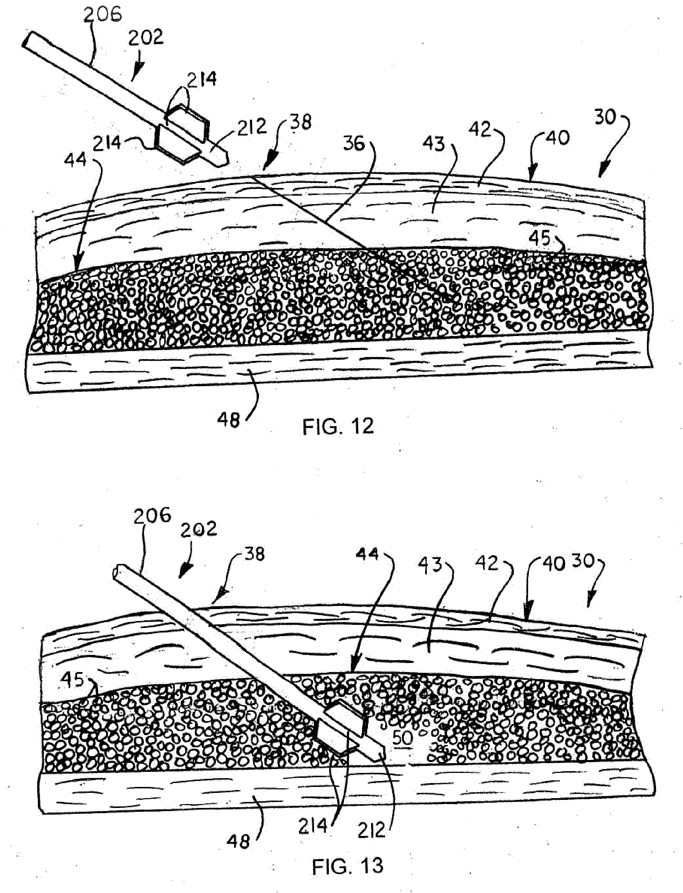

Referring next to FIGS. 12-15 of the drawings, in typical implementation of the tissue eradication methods, the system 201 (FIG. 9) may be used to volumetrically reduce and smoothen the contour of the skin 40 of a patient 30 as part of a cosmetic procedure. In the non-limiting example illustrated in FIGS. 12-15, the method may be implemented in a cosmetic procedure to eliminate or reduce the quantity or volume of adipose cells 45 in an adipose tissue layer 44 at a treatment site 38 in the skin 40 of the patient 30. Accordingly, the cold temperature source 210 (FIG. 9) may be operated to reduce the temperature of the device shaft 206 of the tissue eradication device 202 to the selected target temperature or target temperature range. In some applications, the temperature of the device shaft 206 may be reduced to a target temperature or target temperature range of from about -21.12° C to about 0°C, for example and without limitation.

As illustrated in FIG. 12, as the patient 30 typically lies in a supine or lateral position, an incision 36 may be formed in the skin 40 at or adjacent to the treatment site 38. The incision 36 may extend through the epidermis 42 and dermis 43 and into the underlying adipose tissue layer 44 of the skin 40.

As illustrated in FIG. 13, the device shaft 206 of the tissue eradication device 202 may next be inserted through the incision 36 until the distal shaft end 212 and tissue separating members 214 of the device shaft 206 are disposed within the adipose tissue layer 44. In some applications, the tissue separating members 214 on the cannulated device shaft 206 may separate adipose cells 45 in the adipose tissue layer 44. The supercooled device shaft 206 contacts and induces apoptosis in the adipose cells 45, forming a tissue eradication space 50 in the adipose tissue layer 44.

In some applications, the vibratory motor 204 (FIG. 9) of the tissue eradication device 202 may simultaneously be operated to vibrate or reciprocate the device shaft 206 and achieve enhanced dispersal and contact of the device shaft 206 with the adipose cells 45 within the adipose tissue layer 44 and corresponding enlargement of the tissue eradication space 50. The various operational parameters of the vibratory motor 204, such as the vibration frequency and/or the vibration amplitude, for example and without limitation, may be selected by input from the control unit 205. A typical vibration or reciprocation frequency for the device shaft 206 may fall within the range of about 2,000-10,000 cycles/min, with a typical frequency of about 7,000 cycles/min.

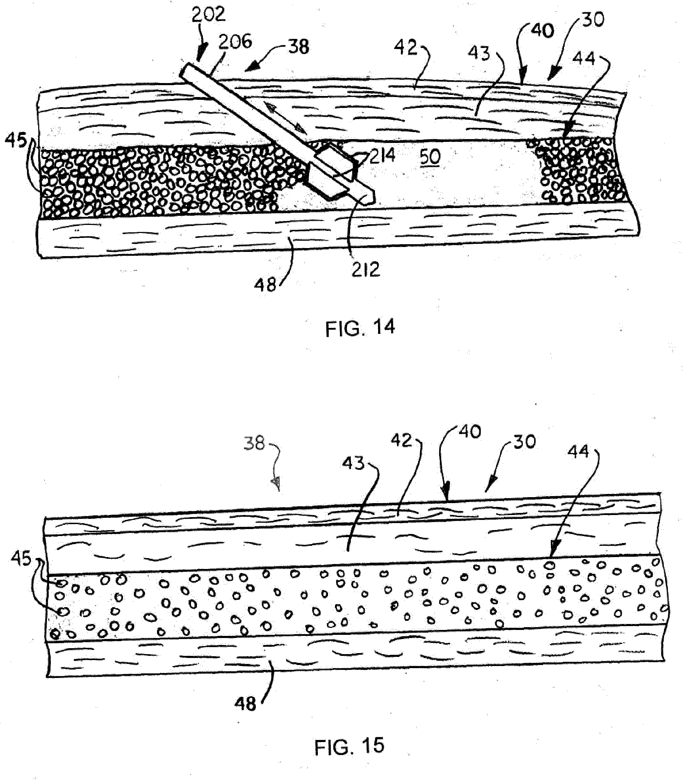

Continued operation of the cold temperature source 210, along with manual movement of the tissue eradication device 202 and vibration of the device shaft 206, may facilitate further inducement of apoptosis in an additional number, quantity or volume of adipose cells 45 and correspondingly enlarging the tissue eradication space 50, as illustrated in FIG. 14. The manual and vibratory movement of the device shaft 206 may be selected depending on the quantity or volume of adipose or tissue which is to be at least partially eradicated from the adipose tissue layer 44 in order to achieve the desired reduction of adipose tissue and cosmetic effect in the skin 40 of the patient 30.

At the conclusion of the tissue eradication procedure, operation of the cold temperature source 210 may be terminated. The device shaft 206 of the tissue eradication device 202 may be removed from the incision 36 and the incision 36 closed. As illustrated in FIG. 15, the adipose cells 45 which remain in the adipose tissue layer 44 may migrate and even out through the adipose tissue layer 44, resulting in a volumetric reduction in the thickness of the skin 40 at and adjacent to the treatment site 38.

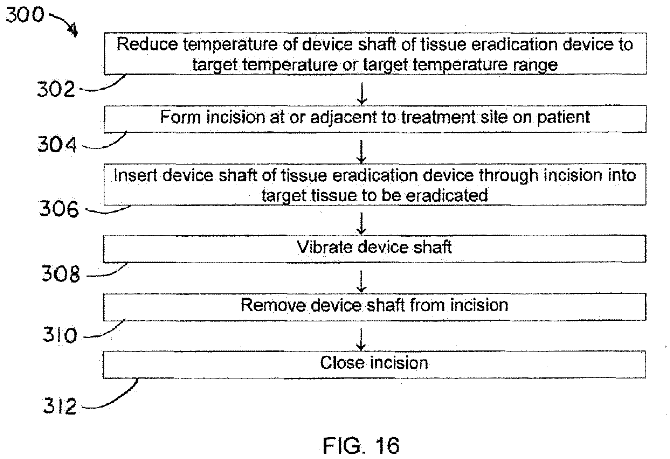

Referring next to FIG. 16 of the drawings, a flow diagram of an alternative illustrative embodiment of the tissue eradication methods using the tissue eradication system 201 illustrated in FIG. 9 is generally indicated by reference numeral 300. At Step 302, the temperature of a device shaft on a tissue eradication device may be reduced to a selected target temperature or target temperature range which is sufficient to induce apoptosis in adipose tissue or other target tissue upon contact of the device shaft with the target tissue. In some applications, the target temperature of the device shaft may fall within a target temperature range of from about -21.12° C to about 0°C.

At Step 304, an incision may be formed at or adjacent to a treatment site on the patient.

At Step 306, the device shaft of the tissue eradication device may be inserted through the incision into the target tissue to be at least partially eradicated.

At Step 308, the device shaft may be vibrated.

At Step 310, the device shaft may be removed from the incision.

At Step 312, the incision may be closed.

While certain illustrative embodiments of the disclosure have been described above, it will be recognized and understood that various modifications can be made to the embodiments and the appended claims are intended to cover all such modifications which may fall within the spirit and scope of the disclosure.

## Claims

1. A tissue eradication system (1) for at least partially eradicating a target tissue, comprising:
a tissue eradication device (2) including:
a gripping portion (3);
a cannulated device shaft (6) extending from the gripping portion (3); and
a cold temperature source (10) including:
a pump (22) disposed in fluid communication with the cannulated device shaft (6);
a cold fluid container (24) disposed in fluid communication with the pump (22), the cold fluid container (24) configured to contain a supply of cold fluid (54); and
a fluid temperature control (26) disposed in thermal communication with the cold fluid container (24).

2. The tissue eradication system (1) of claim 1, further comprising a vibratory motor (4) disposed in contact with at least one of the gripping portion (3) and the cannulated device shaft (6) for vibration of the cannulated device shaft (6).

3. The tissue eradication system (1) of claim 1 or 2, wherein the cannulated device shaft (6) comprises an elongated cannula wall (7), a cannula lumen (8) formed by the cannula wall (7), a distal shaft end (12) having a shaft tip (13), a plurality of tissue separating members (14) extending outwardly from and in spanning relationship between the cannula wall (7) and the distal shaft end (12) and a plurality of fluid flow spaces (18) formed by and between the plurality of tissue separating members (14) and communicating with the cannula lumen (8).

4. A tissue eradication system (1) for at least partially eradicating a target tissue, comprising:
a tissue eradication device (2) including:
a gripping portion (3);
a device shaft extending from the gripping portion (3);
a cold temperature source (16) disposed in thermal contact with the device shaft, the cold temperature source configured to reduce the temperature of the device shaft to a selected target temperature or target temperature range; and
a vibratory motor (4) disposed in contact with at least one of the gripping portion (3) and the device shaft for vibration of the device shaft.

5. The tissue eradication system of claim 4, wherein the cold temperature source (10) comprises a cold fluid container (24) configured to contain a supply of cold fluid (54).

6. A tissue eradication method for at least partially eradicating a target tissue, comprising:
reducing the temperature of a cold fluid (54) to a target temperature or target temperature range;
inserting a cannulated device shaft (6) of a tissue eradication device (2) into the target tissue; and
inducing apoptosis in the target tissue by injecting the cold fluid (54) from the cannulated device shaft (6) of the tissue eradication device (2) into the target tissue.

7. The tissue eradication method of claim 6, further comprising vibrating the cannulated device shaft (6) of the tissue eradication device (2) simultaneous with injecting the cold fluid (54) from the cannulated device shaft (6) of the tissue eradication device (2) into the target tissue., preferably wherein vibrating the cannulated device shaft of the tissue eradication device comprises vibrating the cannulated device shaft at a vibration or reciprocation frequency within a range of about 2,000-10,000 cycles/min, more preferably at the vibration or reciprocation frequency of about 7,000 cycles/min.

8. The tissue eradication method of claim 6 or 7, wherein reducing the temperature of the cold fluid (54) to the target temperature or target temperature range comprises reducing the temperature of the cold fluid (54) to the target temperature or target temperature range of from about -21.12° C to about 0°C.

9. The tissue eradication method of any of claims 6 to 8, wherein injecting the cold fluid (54) from the cannulated device shaft (6) of the tissue eradication device (2) into the target tissue comprises injecting tumescent fluid, hypertonic saline solution, epinephrine solution, liposuction infiltration fluid or lactated ringer's solution from the cannulated device shaft into the target tissue.

10. The tissue eradication method of any of claims 6 to 9, wherein inserting the cannulated device shaft (6) of the tissue eradication device (2) into the target tissue comprises inserting the cannula device shaft (6) having an expanded basket cannula with a plurality of outwardly-extending tissue separation members (14) into the target tissue.

11. The tissue eradication method of any of claims 6 to 10, further comprising providing a pump (22) in fluid communication with the cannulated device shaft (6) of the tissue eradication device (2), providing a cold fluid container (24) in fluid communication with the pump (22) and providing a supply of the cold fluid (54) in the cold fluid container (24), and wherein injecting the cold fluid (54) from the cannulated device shaft (6) of the tissue eradication device (2) into the target tissue comprises pumping the cold fluid (54) from the cold fluid container (24) through the cannulated device shaft (6) into the target tissue.

12. A tissue eradication method for at least partially eradicating a target tissue, comprising:
reducing the temperature of a device shaft on a tissue eradication device (2) to a target temperature or target temperature range;
vibrating the device shaft; and
inducing apoptosis in the target tissue by contacting the target tissue with the device shaft.

13. The tissue eradication method of claim 12, wherein vibrating the device shaft comprises vibrating the device shaft at a vibration or reciprocation frequency within a range of about 2,000-10,000 cycles/min, preferably at the vibration or reciprocation frequency of about 7,000 cycles/min.

14. The tissue eradication method of claim 12 or 13, wherein reducing the temperature of the device shaft on the tissue eradication device (2) to the target temperature or target temperature range comprises reducing the temperature of the device shaft on the tissue eradication device (2) to the target temperature range of from about -21.12° C to about 0°C.

15. The tissue eradication method of any of claims 12 to 14, wherein reducing the temperature of the device shaft on the tissue eradication device (2) to the target temperature or target temperature range comprises placing at least one cold temperature source in thermal communication with the device shaft, preferably wherein placing at least one cold temperature source in thermal communication with the device shaft comprises placing a cold fluid in thermal communication with the device shaft; or
wherein reducing the temperature of the device shaft on the tissue eradication device (2) to the target temperature or target temperature range comprises reducing the temperature of the device shaft having an expanded basket cannula with a plurality of outwardly-extending tissue separation members (14) to the target temperature or target temperature range.
